# EUROPÄISCHE PATENTSCHRIFT

(11) **EP 2 310 783 B1**
(45) Veröffentlichungstag und Bekanntmachung des Hinweises auf die Patenterteilung: **30.03.2016**
(21) Anmeldenummer: 09780279.7
(22) Anmeldetag: 07.07.2009
(51) Int. Cl.: G01N 21/35, G01N 21/47, G01N 21/84, G01N 33/32, F26B 25/22, G01N 21/27

(54) **VERFAHREN ZUR BERÜHRUNGSLOSEN ERMITTLUNG DES TROCKNUNGSGRADES EINER LACKSCHICHT AUF EINER AUSSENHAUT EINES FLUGZEUGS**
METHOD FOR THE CONTACT-LESS DETECTION OF THE DEGREE OF DRYNESS OF A COAT OF VARNISH ON THE EXTERIOR SKIN OF AN AIRCRAFT
PROCÉDÉ POUR DÉTERMINER SANS CONTACT LE DEGRÉ DE SÉCHAGE D'UNE COUCHE DE VERNIS SUR LA PEAU EXTERIEUR D'UN AVION

(30) Priorität: 06.08.2008 DE 102008041052; 06.08.2008 US 188099 P
(43) Veröffentlichungstag der Anmeldung: 20.04.2011
(73) Patentinhaber: Airbus Operations GmbH, 21129 Hamburg (DE)
(72) Erfinder: BENSE, Rolf, 21635 Jork (DE); SIEWERT, Rebecca, 22049 Hamburg (DE)
(74) Vertreter: Isarpatent
(86) Internationale Anmeldenummer: PCT/EP2009/058627
(87) Internationale Veröffentlichungsnummer: WO 2010/015475

(56) Entgegenhaltungen:
- WO-A-03/034042
- DE-A1- 19 737 785
- JP-A- 5 045 288
- JP-A- H02 228 559
- JP-A- S62 103 540
- JP-A- 2002 039 723
- JP-A- 2002 273 308
- JP-A- 2005 172 646
- US-A1- 2005 075 463
- DAY DAVID R ET AL: "In-process endpoint determination of hercules 3501-6" INTERNATIONAL SAMPE SYMPOSIUM AND EXHIBITION (PROCEEDINGS) 1991 PUBL BY SAMPE, Bd. 36, Nr. pt 1, 1991, Seiten 571-581, XP009124974
- XU JAMES J: "FTIR analysis of polyester enamel cure" PROCEEDINGS OF THE ELECTRICAL/ELECTRONICS INSULATION CONFERENCE 1995 IEEE, 1995, Seiten 263-265, XP002553254

## Beschreibung

Die Erfindung betrifft ein Verfahren zur berührungslosen Ermittlung eines Trocknungsgrades einer Lackschicht auf einer Außenhaut eines Flugzeugs.

Um den zunehmend unterschiedlichen Kundenwünschen hinsichtlich der farblichen Gestaltung von Passagierflugzeugen gerecht zu werden, müssen für die Lackierung der Flugzeuge unterschiedlichste Systeme eingesetzt werden. Die Trocknungszeiten der Lackierung hängen von einer Vielzahl von Parametern ab. Von Bedeutung sind beispielsweise der verwendete Lacktyp, die Lufttemperatur und die Luftfeuchtigkeit in der Lackierhalle. Darüber hinaus spielt auch der chemische Aufbau des eingesetzten Lacks eine entscheidende Rolle, denn die Aushärtezeiten variieren erheblich in Abhängigkeit davon, ob es sich beispielsweise um einen Einkomponenten-Lack oder einen Mehrkomponenten-Lack, um einen wasserlöslichen oder einen lösungsmittelhaltigen Lack handelt.
Die Ermittlung des jeweiligen Aushärtungsgrades einer Lackierung erfolgt heutzutage üblicherweise durch manuelle, mechanische Tests. Hierbei wird ein scharfkantiger Messkörper in die Lackschicht hinein gedrückt. Je größer die Kerbwirkung des Messkörpers in der Lackschicht ist, desto weniger weit ist die Aushärtung der Lackschicht fortgeschritten. Der Hauptnachteil dieser Bestimmungsmethode liegt darin, dass die Integrität der Lackschicht beeinträchtigt wird. Darüber hinaus lässt sich diese Bestimmungsmethode nicht automatisieren und die Messunsicherheit ist relativ hoch.

Um den Fertigungsprozess zu beschleunigen, werden heutzutage Trocknungseinheiten, insbesondere Infrarotstrahler, zur Trocknung der Lacke verwendet, die einen sehr hohen elektrischen Energieeinsatz erfordern. Um die Durchlaufzeiten der lackierten Flugzeuge zu minimieren und den Energieverbrauch zu optimieren, ist es wünschenswert, die Einsatzzeiten der Trocknungseinheiten möglichst kurz zu halten und in Abhängigkeit vom Trocknungsgrad der Lackierung steuerbar zu machen. Dies ist mit der bisher angewendeten manuellen Messmethode nicht erreichbar.

JP2002273308 offenbart ein Verfahren zur berührungslosen Ermittlung eines Trocknungsgrades einer Lackschicht mit einer Vorrichtung, welche mindestens einen Sender für eine elektromagnetische Strahlung, mindestens einen Empfänger für die elektromagnetische Strahlung sowie eine Messeinrichtung aufweist, wobei der Trocknungsgrad der Lackschicht durch die Ermittlung einer zeitabhängigen Absorption der auf das Substrat der Lackschicht abgestrahlten und von dem Substrat reflektierten Strahlung ermittelt wird und wobei mittels der Messeinrichtung eine Steigung einer sich aus der zeitlichen Änderung der Absorption ergebenden Messkurve zur Beurteilung des Trocknungsgrades der Lackschicht ermittelt wird.

DE19737785 offenbart ein Verfahren zur berührungslosen Ermittlung eines Trocknungsgrades einer Lackschicht mit einer Vorrichtung, welche mindestens einen Sender für eine elektromagnetische Strahlung, mindestens einen Empfänger für die elektromagnetische Strahlung sowie eine Messeinrichtung aufweist, wobei die von dem mindestens einen Sender emittierte elektromagnetische Strahlung eine Mikrowellenstrahlung ist, wobei der Trocknungsgrad der Lackschicht durch die Ermittlung einer Absorption der auf das Substrat der Lackschicht abgestrahlten und von dem Substrat reflektierten Mikrowellenstrahlung ermittelt wird.

Aufgabe der Erfindung ist es daher, eine automatische, berührungslose Ermittlung des Trocknungsgrades einer Lackschicht bei einer zugleich hohen Messgenauigkeit zu ermöglichen. Ferner ist es Aufgabe der Erfindung, ein Verfahren zur Durchführung einer Trocknungsgradmessung anzugeben.

Diese Aufgabe wird durch ein Verfahren mit den Merkmalen des Patentanspruchs 1 gelöst.

Dadurch, dass eine Vorrichtung mindestens einen Sender für eine elektromagnetische Strahlung, mindestens einen Empfänger sowie eine Messeinrichtung aufweist, kann die Messung eines Trocknungsgrades einer Lackschicht mit einer hohen Genauigkeit erstmals berührungslos und quantitativ erfolgen. Mittels des Senders wird eine elektromagnetische Strahlung geeigneter Wellenlänge auf die Oberfläche der Lackschicht abgestrahlt. Mittels des Empfängers wird eine Intensität der von der Oberfläche der Lackschicht (diffus) reflektierten elektromagnetischen Strahlung ermittelt und zur weiteren Auswertung an eine Messeinheit weitergeleitet und hieraus der aktuelle Trocknungsgrad der Lackschicht ermittelt.
Erfindungsgemäß arbeitet die Vorrichtung mit einer elektromagnetischen Strahlung im Bereich der Mikrowellenstrahlung mit einer Wellenlänge zwischen 1 mm und 1 m. Prinzipiell kann die Vorrichtung in einem Beispiel, das nicht unter die Ansprüche fällt, auch mit einer elektromagnetischen Strahlung im Bereich der nahen Infrarotstrahlung (NIR) mit einer Wellenlänge zwischen 0,8 µm und 2,5 µm betrieben werden.
In beiden Fällen werden die sich im Verlauf des Trocknungsprozesses innerhalb der Lackschicht ergebenden physikalisch-chemischen Veränderungen erfasst, bei denen es sich beispielsweise um das Herausdiffundieren des jeweiligen Lösungsmittels (Wasser oder chemisches Lösungsmittel), um Polymerisationsprozesse oder dergleichen handeln kann.
Im Fall des Einsatzes von Mikrowellenstrahlung werden die Mikrowellen von einem geeigneten Sender auf die Außenhaut mit der Lackschicht abgestrahlt. Ein Empfänger erzeugt ein Messsignal, welches durch die Reflexion hervorgerufen wird. Das Messsignal beinhaltet Verluste durch die Absorption in der Lackschicht und der Außenhau sowie weitere Verluste, zum Beispiel durch Streueffekte. Wird während der Messung der Lacktrocknung ein Abstand des Senders und des Empfängers zur Lackschicht und eine Strahlungsintensität des Senders konstant gehalten, kann davon ausgegangen werden, dass sich auch die Streuverluste nicht verändern und die Intensität der vom Empfänger aufgenommenen Mikrowellen nur noch von der Absorption innerhalb der Lackschicht abhängig ist. Die Absorption bei einer bestimmten Wellenlänge ist von der elektrischen Permittivität (Dielektrizitätszahl) des Lackes abhängig, die wiederum mit dem Trocknungsgrad des Lacks korreliert. Da zwischen der Permittivität und der Trocknung ein Zusammenhang besteht, kann über die Messung der Absorption der jeweilige Trocknungsgrad ermittelt werden. Auf die Kenntnis der absoluten Intensität der Mikrowellenstrahlung kommt es hingegen nicht an, da nur eine zeitliche Änderung der vom Empfänger aufgenommenen Mikrowellenstrahlung bei gleich bleibender Ausgangsintensität und bei auch ansonsten konstanten Umgebungsbedingungen durch die Ermittlung der ersten Ableitung des gemessenen Verlaufs der Mikrowellenstrahlung über die Zeit ausgewertet wird.

Beim nicht unter die Ansprüche fallenden Einsatz von Infrarotstrahung hingegen werden die Molekülbindungen im Lack zu Schwingungen angeregt. Unterschiedliche Molekülbindungen benötigen eine unterschiedliche Energie (Frequenz, W = h* f), um überhaupt angeregt zu werden. Hieraus folgt, dass jeweils bestimmte Molekülbindungen nur durch bestimmte Frequenzen angeregt werden können. Durch die Anregung wird die eingestrahlte Energie im Lack in Bewegungsenergie (Wärme) umgewandelt und somit letztendlich absorbiert. Daraus folgt, dass bestimmte Molekülgruppen jeweils charakteristische Wellenlängen absorbieren. Zur Überwachung des Trocknungsverlaufs eines Lackes mittels elektromagnetischer Strahlung im nahen Infrarotbereich ist daher die Kenntnis der sich während der Trocknung verändernden chemischen Verbindungen im Lack erforderlich. Ein Beispiel hierfür ist Wasser, das sich insbesondere bei wasserlöslichen Lacken während der Trocknung verflüchtigt. Aus der Messung der von Wassermolekülen typischerweise absorbierten Frequenzen bzw. Wellenlängen lässt sich eine Messkurve ermitteln, bei der die Reflexion dieser Frequenzen über die Zeit bis zu einem Maximum zunimmt. Im Fall von anderen Molekülgruppen kann sich auch eine Zunahme der Absorption über die Zeit einstellen, wenn zum Beispiel bei der Aushärtung von Mehrkomponentenlacken neue Molekülgruppen gebildet werden. Abhängig vom jeweils konkret verwendeten Lacksystem gibt es jedoch immer Frequenzen, bei denen eine zeitliche Änderung der Reflexion über die relevante Trocknungsdauer messbar ist.
Die Kenntnis von absoluten Strahlungsintensititäten ist hingegen beim nicht unter die Ansprüche fallenden Einsatz von Infrarotstrahlung irrelevant, denn durch die Vorrichtung wird nur eine Steigung der sich aus dem zeitlichen Verlauf der Reflexionsmessungen ergebenden Messkurve ausgewertet.

Die Messeinheit kann mit einer Steuer- und/oder Regeleinrichtung gekoppelt sein. Hierdurch kann der gesamte Mess- und Auswertungsablauf innerhalb der Vorrichtung automatisiert werden. Zudem ermöglicht die kombinierte Steuer-und/oder Regeleinrichtung eine selbsttätige Ansteuerung weiterer Funktionsgruppen innerhalb der Vorrichtung.

Nach Maßgabe einer weiteren vorteilhaften Ausgestaltung ist vorgesehen, dass mindestens eine Trocknungseinrichtung, insbesondere mindestens ein elektrischer Infrarotstrahler, vorgesehen ist, die in Abhängigkeit von dem erreichten Trocknungsgrad der Lackschicht durch die Steuer- und/oder Regeleinrichtung steuerbar, insbesondere beim Erreichen eines vorgesehenen Trocknungsgrades selbsttätig abschaltbar ist.
Hierdurch ist eine selbsttätig arbeitende Trocknungseinrichtung realisierbar, der eine vollautomatische Lackiereinrichtung vorgeschaltet ist.

Durch die Verfahren ist erstmals eine relativ genaue, berührungslose und daher zerstörungsfreie Ermittlung des Aushärtungsgrades einer auf eine Außenhaut aufgetragenen Lackschicht möglich, wodurch zudem erstmalig die Integrierbarkeit in vollautomatische Lackier- und Trocknungssysteme gegeben ist.

In der Zeichnung zeigt:
- **Fig. 1**: Ein Diagramm, das einen Verlauf des Trocknungsgrades einer Lackschicht in Prozent aufgetragen über die verstrichene Trocknungszeit in Minuten zeigt,
- **Fig. 2**: einen schematischen Aufbau der Vorrichtung bei der Verwendung von elektromagnetischer Strahlung im Mikrowellenbereich,
- **Fig. 3**: eine mit der Vorrichtung beim nicht unter die Ansprüche fallenden Einsatz von elektromagnetischer Strahlung im nahen Infrarotbereich (NIR) ermittelte Messkurve, und
- **Fig. 4**: einen aus der Messkurve nach Fig. 3 ermittelten zeitlichen Verlauf der Absorption A(λ,t) bei einer Wellenlänge von λ =1480 nm und eine hierfür numerisch berechnete Annäherungskurve.

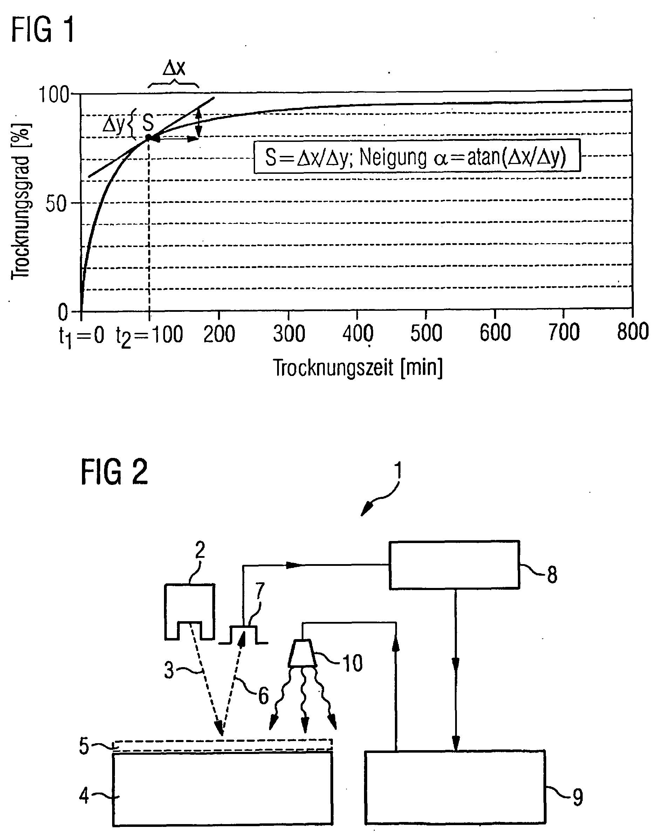

Das Diagramm in Fig. 1 zeigt zunächst den zeitlichen Verlauf eines exemplarischen Trocknungsprozesses einer Lackschicht, wie er mittels der Vorrichtung ermittelbar ist.
Auf der vertikalen Ordinate des Diagramms ist der jeweils erreichte Trocknungsgrad in Prozent abgetragen, während auf der horizontalen Abszisse die bisher abgelaufene Trocknungszeit in Minuten dargestellt ist. Deutlich ist zu erkennen, dass bereits zum Zeitpunkt t₂ = 100 Minuten, das heißt wenn eine Steigung S, das heißt die Ableitung der Trocknungskurve nach der Zeit t, einen Wert von etwa 0,6 (entspricht 35°) erreicht hat, ein Trocknungsgrad von nahezu 80 Prozent erreicht ist. Hieraus folgt, dass eine weitere Ausdehnung der Trocknungsdauer t₂-t₁, nachdem die Messkurve bei t₂ = 100 Minuten eine Steigung S von 0,6 unterschreitet, im Allgemeinen nicht mehr sinnvoll ist. In der Regel ist bereits ein Wert von S ≤ 0,3 bei den im Flugzeugbau eingesetzten Lacksystemen ein geeignetes Abbruchkriterium zur automatischen Steuerung des Trocknungsprozesses.

Die **Fig. 2** zeigt den prinzipiellen Aufbau einer Vorrichtung zur Ermittlung des aktuellen Trocknungs- bzw. Aushärtungsgrades einer auf eine Außenhaut aufgebrachten Lackschicht.

Die Vorrichtung 1 umfasst einen Sender 2, der in einer ersten Ausführungsvariante elektromagnetische Strahlung im Mikrowellenbereich abgibt. Der Sender 2 umfasst im Allgemeinen eine nicht dargestellte Ansteuerelektronik sowie einen hiermit verbundenen, für die jeweilige Wellenlänge der abzugebenden elektromagnetischen Strahlung geeigneten Strahler.
Der Sender 2 strahlt Mikrowellenstrahlung 3 auf eine auf eine Außenhaut 4 aufgebrachte Lackschicht 5 ab. Die von der Lackschicht 5 reflektierte Mikrowellenstrahlung 6 wird von einem für die jeweils eingesetzte elektromagnetische Strahlung geeigneten Empfänger 7, also im Fall von Mikrowellenstrahlung beispielsweise von einer Antenne aufgefangen, und an eine nachgeschaltete Messeinrichtung 8 zur Verstärkung, Aufbereitung (Filterung etc.), Digitalisierung und numerischen Auswertung der von der Antenne 7 aufgefangenen Rohmesswerte weitergeleitet. In der Messeinrichtung 8 erfolgt die Messung der vom Empfänger aufgenommenen und von der Lackschicht 5 und/oder der Außenhaut 4 reflektierten Mikrowellenstrahlung 6. In der Messeinrichtung 8 erfolgt vorzugsweise bereits eine rechnerische Aufbereitung der Rohmesswerte. Die in der Messeinrichtung 8 verstärkten, aufbereiteten und ausgewerteten Messwerte können unmittelbar mittels einer Ausgabeeinrichtung visualisiert und/oder abschließend an eine Steuer- und Regeleinrichtung 9 weitergeleitet werden, um beispielsweise eine Trocknungseinrichtung selbsttätig beim Erreichen eines vorgewählten Trocknungsgrades abzuschalten. Beispielsweise kann in der Messeinrichtung 8 der zeitliche Verlauf der Absorption A(λ,t) bei einer bestimmten Wellenlänge bzw. Frequenz (*λ * f = c)* auf einem geeigneten Anzeigegerät, Display, Monitor, Bildschirm oder dergleichen grafisch dargestellt werden.
Eine geeignete Trocknungseinrichtung ist bevorzugt mit einer Vielzahl von elektrisch betriebenen, matrixförmig angeordneten Infrarotstrahlern 10 aufgebaut, um eine großflächige und möglichst gleichmäßige Temperierung der Lackschicht 5 zur Beschleunigung des Trocknungsprozesses zu erreichen. Die Messeinrichtung 8 und die Steuer- und Regeleinrichtung 9 können Bestandteile einer integrierten Rechnereinheit, insbesondere eines herkömmlichen Personalcomputers (z.B. ein PC), sein.

Die vom Sender 2 emittierte Mikrowellenstrahlung 3 wird in der Lackschicht 5 und/oder von der Außenhaut 4 in Abhängigkeit von der aktuellen Permittivität, die wiederum vom Aushärtungsgrad der Lackschicht 5 abhängt, absorbiert. Die von der Lackschicht 5 bzw. der Außenhaut 4 nicht absorbierte bzw. der reflektierte Anteil der Mikrowellenstrahlung 6 wird anschließend vom Empfänger 7 gemessen. Der Strahler des Senders 2 für Mikrowellen und die Antenne als Empfänger 7 können als ein integrales Bauteil ausgeführt sein. Aus der Differenz zwischen der in der Regel bekannten Intensität der vom Sender 2 abgegebenen Mikrowellenstrahlung 3 und der vom Empfänger 7 ermittelbaren Intensität der reflektierten Mikrowellenstrahlung 6 wird in der Messeinrichtung 8 eine Absorption A(t) ermittelt, die mit dem Verlauf des Trocknungsgrades der Lackschicht 5 in hohem Maße korreliert. Mittels der Steuer- und/oder Regeleinrichtung 9 kann dann beispielsweise eine Abschaltung einer mit einem oder mehreren Infrarotstrahlern 10 gebildeten Trocknungseinrichtung vollzogen werden, wenn zu einem fraglichen Trocknungszeitpunkt t₁, also beispielsweise 100 Minuten nach dem zeitlichen Beginn tₒ des Trocknungsvorgangs, die Steigung S der ermittelten Messkurve (vgl. Fig. 1) beispielsweise kleiner oder gleich dem Wert 0,3 wird, da in diesem Fall auch durch eine überproportionale Ausdehnung der Aushärtungszeit kein nennenswert höherer Trocknungsgrad mehr erreichbar ist. Die Steigung S entspricht der Neigung einer im fraglichen Trocknungszeitpunkt t₁ an die Messkurve angelegten Tangente. Zusätzlich kann auch die Wellenlänge der Mikrowellenstrahlung variiert werden, um einen wellenlängen- und zeitabhängigen Verlauf der Absorption A(λ,t) zu bestimmen.

In einem in der Zeichnung nicht dargestellten und nicht unter die Ansprüche Fallenden Beispiel wird die Vorrichtung 1 mit einer elektromagnetischen Strahlung im so genannten nahen Infrarotbereich bzw. Infrarotspektrum zwischen 0,8 µm und 2,5 µm betrieben (so genannte NIR-Strahlung). Hierbei findet als Messverfahren das diffuse Reflexionsmessverfahren Anwendung. In diesem Beispiel kann der Sender 2 beispielsweise mit einer oder mehreren Halbleiter-IR-Dioden aufgebaut sein, die elektromagnetische Strahlung mit einer konstanten Wellenlänge im nahen Infrarotbereich auf die Lackschicht 5 abstrahlen. Der Empfänger 7 umfasst zum Beispiel mindestens eine Fotodiode zur Auswertung der von der Lackschicht 5 bzw. der Außenhaut 4 reflektierten Infrarotstrahlung, wobei die vom Empfänger aufgenommene diffuse IR-Strahlung in ein elektrisches Ausgangssignal umgewandelt wird, das heißt in der Messeinrichtung 8 entsprechend aufbereitet, das heißt insbesondere gefiltert, verstärkt, digitalisiert und numerisch durch eine Ableitung nach der Zeit aufbereitet wird. Die interessierende zeitliche Änderung der Absorption A(λ,t) lässt sich aus dem vom Empfänger 7 generierten Messsignal bei der jeweils untersuchten Wellenlänge ermitteln. Eine genaue Kenntnis der Intensität der vom Sender 2 emittierten elektromagnetischen Strahlung im Wellenlängen- bzw. Frequenzbereich des nahen Infrarots ist nicht nötig, denn die Lacktrocknung kann zum Beispiel bei einer Steigung S ≤ 0,3 der Messkurve (vgl. Fig. 1) in der Regel als beendet angesehen werden. Auf eine Absolutwertmessung kommt es daher in diesem Zusammenhang nicht an.

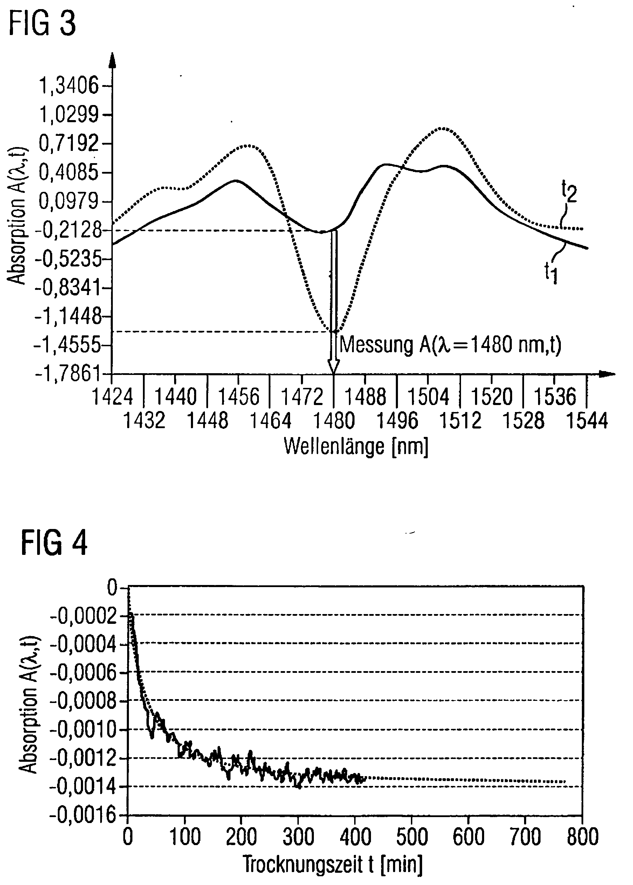

Die vom Sender 2 abgegebene nahe Infrarotstrahlung bzw. IR-Strahlung liegt in einem Energiebereich der Schwingungsniveaus der in der Lackschicht 5 enthaltenen Moleküle, das heißt die Absorption der IR-Strahlung führt zu einer entsprechenden Schwingungsanregung der intermolekularen Bindungen. Diese Schwingungszustände werden im gemessenen Absorptionsspektrum A(λ,t) in Form von Spitzen (so genannte "Peaks") bzw. Senken, wie im Diagramm in **Fig. 3** exemplarisch dargestellt, sichtbar. Da die zugehörigen Energien bzw. Frequenzen der Strahlung charakteristisch für die jeweils vorherrschenden Bindungen sind, können bestimmte chemische Zusammensetzungen bzw. Moleküle identifiziert werden. Da sich die Beschaffenheit bzw. Zusammensetzung der Lackschicht 5 infolge des Trocknungsprozesses aufgrund der hierbei ablaufenden, komplexen chemisch-physikalischen Prozesse kontinuierlich verändert, diese Variationen der Zusammensetzung und der Inhaltsstoffe sich in einer Veränderung des gemessenen IR-Absorptionsspektrums niederschlagen, lässt sich hieraus der jeweils aktuelle Aushärtungsgrad bzw. Trocknungsfortschritt der Lackschicht 5 auf der Außenhaut 4 ableiten.

Die in den **Fig. 3,4** dargestellten Messkurven für die Absorption A(λ,t) geben lediglich deren qualitativen Verlauf wieder und sind nicht zur Bestimmung quantitativer Absorptionswerte gedacht.
Als vorteilhaft für die Ermittlung des Trocknungsgrades aktuell im Flugzeugbau eingesetzter Lacke bzw. Lacksysteme hat sich, wie die mit der vorstehend erläuterten Vorrichtung 1 ermittelte Messkurve nach **Fig. 3** zeigt, zum Beispiel die Messung des Verlaufs der Absorption A(λ,t) bei einer Wellenlänge der IR-Strahlung von etwa λ=1480 nm herausgestellt. Die Wellenlänge von λ=1480 nm ist insbesondere für die in der Lackschicht 5 enthaltenen Urethangruppen (-CO-NH-R) typisch und wird zudem von keiner störenden Absorption der Ausgangsstoffe in der Lackschicht 5 und/oder des Lösungsmittels bzw. des Verdünnungsmittels überlagert. Deutlich ist in der Messkurve zu erkennen, dass ausgehend von einem Startzeitpunkt des Trocknungsprozesses t₁ bei 0 Minuten bis zum Endzeitpunkt t₂ des Trocknungsprozesses bei beispielsweise 800 Minuten der Absorptionswert A(λ,t) von einem mittleren Ausgangswert für A(λ,t) steil bis auf einen Minimalwert abfällt. Bei anderen Lacksystemen tritt die zur Auswertung relevante zeitliche Änderung der Absorption A(λ,t) im Allgemeinen aufgrund einer abweichenden chemischen Zusammensetzung bei anderen Wellenlängen auf. So ist beispielsweise im Fall von wasserbasierten Lacksystemen die Absorptionswellenlänge von Wasser bzw. Wasserdampf maßgeblich.

Im Ergebnis korreliert der Verlauf des Absorptionswertes A(λ,t) bei λ = 1,48 µm sehr gut mit dem Fortschritt des Trocknungsprozesses innerhalb der Lackschicht 5 auf der Außenhaut 4, so dass die bei dieser Wellenlänge ermittelten Absorptionswerte A(λ=1480 nm,t) - nach gegebenenfalls erforderlichen numerischen Zwischenoperationen innerhalb der Messeinrichtung 8 - in vorteilhafter Weise als ein Maß für den jeweils aktuellen Trocknungsgrad der Lackschicht 5 herangezogen werden können.

Die in **Fig. 4** mit einer durchgezogenen Linie gezeichnete Messkurve stellt die mittels der Vorrichtung 1 gemessene Absorption A(λ,t) über den gesamten untersuchten Trocknungszeitraum t der Lackschicht 5 zwischen beispielsweise t₁ = 0 Minuten und t₂ = 800 Minuten bei einer festen Infrarotwellenlänge von 1480 nm dar A(λ=1480 nm,t). Die punktiert dargestellte Linie verkörpert eine mittels bekannter mathematischer Methoden gewonnene numerische Näherungskurve für die Messkurve der gemessenen Absorption A(λ,t). Wie zu erkennen, ist diese Näherungskurve die Inverse der Kurve nach Fig. 1, aus der unmittelbar der Trocknungsgrad der Lackschicht 5 in Prozent über die Trocknungszeit t in Minuten abgelesen werden kann. Somit lässt sich mittels Inversion aus der Rohmesskurve nach Fig. 4 die Näherungskurve nach Fig. 4 ermitteln, aus der direkt die Trocknungskurve nach Fig. 1 ableitbar ist.

Die der Trocknungskurve nach Fig. 1 entnehmbaren Werte können im Bedarfsfall unmittelbar zu Anzeigezwecken für einen Anwender der Vorrichtung 1 benutzt werden.

Zur Ansteuerung einer aktiven Trocknungseinrichtung mit Infrarotstrahlern 10 ist jedoch die Verfolgung der Steigung S der Messkurve nach Fig. 1, das heißt lediglich die zeitliche Änderung der Absorption A(λ ,t) im nahen Infrarotbereich bei einer bestimmten Wellenlänge ausreichend, so dass beispielsweise bei Steigungswerten S ≤ 0,3 die Infrarotstrahler 10 mittels der Steuer- und Regeleinrichtung 9 abgeschaltet werden können.

### Bezugszeichenliste

- 1: Vorrichtung
- 2: Sender (NIR/Mikrowelle)
- 3: Mikrowellenstrahlung (ausgestrahlt)
- 4: Außenhaut (z.B. Rumpfzellenhaut)
- 5: Lackschicht
- 6: Mikrowellenstrahlung (reflektiert)
- 7: Empfänger
- 8: Messeinrichtung
- 9: Steuer- und/oder Regeleinrichtung
- 10: Infrarotstrahler

## Patentansprüche

1. Verfahren zur berührungslosen Ermittlung eines Trocknungsgrades einer Lackschicht (5) auf einer Außenhaut (4) eines Flugzeugs mit einer Vorrichtung (1), welche mindestens einen Sender (2) für eine elektromagnetische Strahlung, mindestens einen Empfänger (7) für die elektromagnetische Strahlung sowie eine Messeinrichtung (8) aufweist, wobei die von dem mindestens einen Sender (2) emittierte elektromagnetische Strahlung eine Mikrowellenstrahlung (3) ist, insbesondere mit einer konstanten Wellenlänge zwischen 1 mm und 1 m, wobei der Trocknungsgrad der Lackschicht (5) durch die Ermittlung einer zeitabhängigen Absorption (A) der auf die Außenhaut (4) abgestrahlten und von der Außenhaut (4) reflektierten Mikrowellenstrahlung (3) bei mindestens einer konstanten Wellenlänge (λ) der Mikrowellenstrahlung (3) ermittelt wird und wobei mittels der Messeinrichtung (8) eine Steigung einer sich aus der zeitlichen Änderung der Absorption (A) ergebenden Messkurve zur Beurteilung des Trocknungsgrades der Lackschicht (5) ermittelt wird.

2. Verfahren nach Patentanspruch 1, **dadurch gekennzeichnet, dass** die Auswertung der Absorption (A) über ein Zeitintervall hinweg, insbesondere über einen Trocknungszeitraum von bis zu 48 h, erfolgt.

3. Verfahren nach einem der vorhergehenden Patentansprüche, **dadurch gekennzeichnet, dass** die Messeinrichtung (8) mit einer Steuer- und/oder Regeleinrichtung (9) gekoppelt ist und mindestens eine Trocknungseinrichtung (10) vorgesehen ist, die in Abhängigkeit von dem erreichten Trocknungsgrad der Lackschicht (5) durch die Steuer- und/oder Regeleinrichtung (9) gesteuert wird und beim Erreichen eines vorgewählten Trocknungsgrades selbsttätig abgeschaltet wird.

## Claims

1. Method for contactless detection of the level of dryness of a coat of varnish (5) on the outer skin (4) of an aircraft, comprising a device (1) which has at least one transmitter (2) for electromagnetic radiation, at least one receiver (7) for the electromagnetic radiation and a measurement device (8), the electromagnetic radiation emitted by the at least one transmitter (2) being microwave radiation (3), in particular having a constant wavelength between 1 mm and 1 m, the level of dryness of the layer of varnish (5) being detected by detecting the time-dependent absorption (A) of the microwave radiation (3) radiated onto the outer skin (4) and reflected by the outer skin (4) at at least one constant wavelength (λ) of the microwave radiation (3), and an increase in a measurement curve, which results from the change over time in the absorption (A), being detected by the measurement device (8) to assess the level of dryness of the coat of varnish (5).

2. Method according to claim 1, **characterised in that** the absorption (A) is evaluated over a time interval, in particular over a drying period of up to 48 h.

3. Method according to either of the preceding claims, **characterised in that** the measurement device (8) is coupled to a control and/or regulation device (9), and at least one drying device (10) is provided, which is controlled by the control and/or regulation device (9) as a function of the achieved level of dryness of the coat of varnish (5) and is switched off automatically when a preselected level of dryness is achieved.

## Revendications

1. Procédé pour déterminer sans contact le degré de séchage d'une couche de vernis (5) sur l'enveloppe externe (4) d'un avion comportant un dispositif (1) qui présente au moins un émetteur (2) pour un rayonnement électromagnétique, au moins un récepteur (7) pour le rayonnement rayonnement électromagnétique ainsi qu'un moyen de mesure (8), le rayonnement électromagnétique émis par au moins un émetteur (2) étant un rayonnement hyperfréquence (3), en particulier avec une longueur d'onde constante entre 1 mm et 1 m, le degré de séchage de la couche de vernis (5) étant déterminé en déterminant une absorption (A) en fonction du temps du rayonnement hyperfréquence (3) émis sur l'enveloppe externe (4) et réfléchi par l'enveloppe externe (4) à au moins une longueur d'onde (λ) constante du rayonnement hyperfréquence (3) et une pente d'une courbe de mesure résultant de l'évolution temporelle de l'absorption (A) étant déterminée au moyen du moyen de mesure (8) pour évaluer le degré de séchage de la couche de vernis (5).

2. Procédé selon la revendication 1, **caractérisé en ce que** l'analyse de l'absorption (A) est effectuée durant un intervalle de temps, en particulier pendant une durée de séchage allant jusqu'à 48 heures.

3. Procédé selon l'une des revendications précédentes, **caractérisé en ce que** le moyen de mesure (8) est accouplé à un moyen de commande et/ou de réglage (9) et au moins un moyen de séchage (10) est prévu, qui est commandé en fonction du degré de séchage atteint de la couche de vernis (5) par le moyen de commande et/ou de réglage (9) et est arrêté automatiquement lorsqu'un degré de séchage présélectionné est atteint.
